# EUROPEAN PATENT SPECIFICATION

(11) **EP 1 432 993 B1**
(45) Date of publication and mention of the grant of the patent: **25.04.2012**
(21) Application number: 02782119.8
(22) Date of filing: 04.10.2002
(51) Int. Cl.: A61K 31/327, A61L 2/00, A61L 2/14, C11D 3/39, C11D 11/00, A61L 2/18, A61L 2/20

(54) **In vitro evaluation method for priocidal (anti-prion) treatments**
In vitro Verfahren, um gegen Prionen gerichtete Behandlungen abzuschätzen
Méthode d'évaluation in vitro de traitements anti-prions

(30) Priority: 05.10.2001 US 327460 P
(43) Date of publication of application: 30.06.2004
(73) Proprietor: STERIS INC., Temecula, CA 92590 (US)
(72) Inventor: ANTLOGA, Kathleen, M., Chardon, OH 44024 (US); MCDONNELL, Gerald, E., Basingstoke, Hampshire RG213DP (GB)
(74) Representative: von Kreisler Selting Werner
(86) International application number: PCT/US2002/031872
(87) International publication number: WO 2003/031987

(56) References cited:
- WO-A-00/71575
- WO-A-01/00235
- FR-A- 2 814 177
- US-B2- 7 001 873
- DATABASE BIOSIS [Online] BIOSCIENCES INFORMATION SERVICE, PHILADELPHIA, PA, US; BURDON, D.W. ET AL: "Replication of IFDO on a chemically defined medium" Database accession no. 1996:418621 XP002249888 & JOURNAL OF MEDICAL MICROBIOLOGY, vol. 45, no. 1, 1996, pages 10-15,
- DYAS A.C.; BURDON D.W.: "Studies of a novel agent possessing resistance to moist heat and disinfectants parallels with Creutzfeldt-Jakob agent", JOURNAL OF HOSPITAL INFECTION, vol. 15, no. 3, 1990, pages 265-272, XP008079671, ISSN: 0195-6701
- ANTLOGA K. ET AL: "Prion disease and medical devices", ASAIO JOURNAL, vol. 46, no. 6, 1 November 2000 (2000-11-01), pages S69-S72, XP001092854, USA
- ERNST D.R.; RACE R.E.: "Comparative analysis of scrapie agent inactivation methods", JOURNAL OF VIROLOGICAL METHODS, vol. 41, no. 2, 1 February 1993 (1993-02-01), pages 193-201, XP000865557, NETHERLANDS
- ZOBELEY E. ET AL: "Infectivity of scrapie prions bound to a stainless steel surface.", MOLECULAR MEDICINE (CAMBRIDGE, MASS.), vol. 5, no. 4, 1 April 1999 (1999-04-01), pages 240-243, USA
- BURDON D.W.: "A novel replicating agent isolated from the human intestinal tract having characteristics shared with Creutzfeldt-Jakob and related agents", JOURNAL OF MEDICAL MICROBIOLOGY, vol. 29, no. 2, June 1989 (1989-06), pages 145-157, ENGLAND
- TAYLOR D.M.: "Inactivation of transmissible degenerative encephalopathy agents: A review.", VETERINARY JOURNAL, vol. 159, no. 1, January 2000 (2000-01), pages 10-17, XP008012858, ENGLAND

## Description

### Background of the Invention

The present invention relates to the field of infectious diseases. It finds particular application as a method of evaluating the response of Prions (Proteinaceous Infectious Agents) to various treatments, and will be described with particular reference thereto. It should be appreciated, however, that the invention is also applicable to other studies of prion activity.

The term "Prion" is used to describe proteinaceous-infectious agents that cause relatively similar brain diseases in humans and/or in animals, which are invariably fatal. These diseases are generally referred to as transmissible spongiform encephalopathies (TSEs). TSEs include Creutzfeldt-Jakob disease (CJD) and variant CJD (vCJD) in humans, Bovine Spongiform Encephalopathy (BSE) in cattle, also know as "Mad Cow Disease," Scrapie in sheep, and Wasting Disease in elk. All of these diseases attack the neurological organs of the animal or animals which are susceptible to the particular disease. They are characterized by initially long incubation times followed by a short period of neurological symptoms, including dementia and loss of coordination, and eventually death.

The infectious agent responsible for these diseases is thought to be a simple protein, with no associated nucleic acids. The pathogenic mechanism for such prion diseases is proposed to involve an initially normal host encoded protein. The protein undergoes a conformational change to an abnormal form (a prion), which has the ability of self-propagation. The exact cause of this change is, at present, unknown. The abnormal form of the protein is not broken down effectively in the body and its accumulation in certain tissues (in particular neural tissue) eventually causes tissue damage, such as cell death. Once significant neural tissue damage has occurred, the clinical signs are observed.

Prion diseases may thus be classified as protein aggregation diseases, which also include several other fatal diseases, such as Alzheimer's disease and amyloidosis. In the case of CJD, the most prevalent prion disease in humans (occurring in roughly 1:1,000,000 of the population), about 85% of cases are thought to arise sporadically, about 10% are thought to be inherited, and about 5% arise iatrogenically.

There are currently no known effective treatments for prion diseases in animals or humans, and death thus follows the onset of neurological symptoms. Progress in the identification of target treatment drugs has been slow, due to the inability to perform testing *in vitro.* To date, no methods for culturing prions in media in the laboratory have been developed. *In vivo* studies involve inoculating a test animal with prions and examining the animal's response to a proposed treatment regime. Because progress of the disease is slow, these in vivo studies are inevitably lengthy and are thus not readily amenable to the screening of large numbers of potential drugs. *In vivo* mouse or hamster models have been engineered to be more susceptible to prions and are generally used for evaluations. In addition, because these diseases tend to be animal specific, it is not known whether tests done on animals can be readily applied to humans.

Some research groups have suggested using a yeast prion model for drug evaluation and there has been some reports of an *in vitro* model to study prion folding. However, there have been no studies which have established correlations between the behavior of these proposed models and prion activity.

In the early 1980's, a novel replicating agent was isolated from the human intestinal tract. (Burdon, J. Med. Micro., 29: 145-157 (1989) and Burdon; The Lancet, Vol. 353; April 10 (1999)). This agent was isolated from the ileostomy fluid (filtered through a 0.2*µ* filter) of two patients with Crohn's disease, and could be cultured *in vitro*. It was given the name Ileal Fluid Dependent Organism (IFDO), although it has been subsequently found to survive in other media, such as in the presence of pancreatin. Discrete brown colonies were observed on a specific, select growth media. On examination of this agent, it did not appear to be viral, bacterial, or fungal in nature, but did appear to grow logarithmically and have unusual resistance to a variety of antibiotics, and physical and chemical agents. The agent was also found to have a high resistance to moist heat. This agent has not previously been directly linked to prions or used in prion research.

Although prion diseases have not generally been considered to be highly contagious, they can be transmitted within a species and, under certain conditions, from one species to another. It has recently been shown that prion diseases may be transmitted via high risk tissues, including the brain, spinal cord, and eye. Iatrogenic transmission has also been reported, including transmission via dura mater grafting, corneal transplants, pericardial homografts, human gonadotropin, and human growth hormone contamination. Transmission via medical devices has also been reported, including through reuse of neurosurgical instruments, depth electrodes, and other devices used during surgeries in close proximity to the central nervous system.

There is currently much speculation about the efficacy of conventional decontamination and sterilization methods for destruction of prions. Prions are notoriously very hardy and demonstrate resistance to routine methods of decontamination and sterilization. Some recommended methods include incineration, prolonged steam autoclaving, sodium hydroxide and sodium hypochlorite treatments at high concentrations (e.g., 1M NaOH or NaHClO₃ at 2% available Cl for 1 hr.). These aggressive treatments are often incompatible with medical devices, particularly flexible endoscopes and other devices with plastic, brass, or aluminum parts. Many devices are damaged by exposure to high temperatures. Chemical treatments, such as strong alkali, are damaging to medical device materials or surfaces in general. Glutaraldehyde, formaldehyde, hydrogen peroxide, most phenolics, alcohols, and processes such as dry heat, boiling, freezing, UV, ionizing, and microwave radiation have generally been reported to be ineffective. There is a clear need for products and processes that are effective against prions yet compatible with surfaces.

One less aggressive treatment which has been investigated and shown to be effective against prions is a peracetic acid formulation formulated by STERIS Corporation, Mentor, Ohio, under the tradename STERIS 20™. The formulation contains peracetic acid in a blend of buffers, anticorrosives, surfactants, and chelators, prepared in a use dilution for sterile processing at above room temperature.

However, there is currently no ready means of evaluating anti-prion ("priocidal") treatments. Culturing prion-treated devices after proposed priocidal treatments involves inoculating animals with washings from the devices and observing the development of the disease if the priocidal treatment is ineffective. This is a lengthy process and prone to errors, since the numbers of prions remaining on the devices may be relatively small. Additionally, there is a risk that prions which are not destroyed by the priocidal treatment may pose hazards to workers.

There are thus increased concerns among medical personnel regarding the proper care of patients identified as having prion diseases. There are also concerns that the diseases may be transmitted, through reuse of instruments and the like, due to a failure to detect the disease state prior to death of the infected patient. Additionally, the risks associated with high, medium, and low risk tissues have not yet been established. For example, tonsillectomy and dental procedures have been considered to be low risk procedures for potential prion infection. However, recent evidence suggests the risks may higher, due to the finding that prion infected tissues are being found outside the brain. It has also been suggested that there may be a link between prion-related diseases and similar disease states, such as Parkinson's and Alzheimer's diseases.
"Journal of Hospital Infection", (1990) Vol. 15, No. 3, pp 265-272 (D1) relates to a study of the resistance of a replicating agent (IFDO) having similarities to Creutzfeldt-Jacob agent (CJA). At page 270, D1 notes that IFDO may provide a model for prions, and that the data reported in the study may be relevant to the safe disposal of material infected with Creutzfeldt-Jacob agent. There is no suggestion in D1, however that the IFDO be used to assess a treatment which includes cleaning a substrate with a cleaning composition which removes proteins, and contacting the cleaned substrate with an oxidizing agent.

The present invention provides a new and improved method for evaluation of priocidal activity, which overcomes the above-referenced problems.

### Summary of the Invention

In accordance with one aspect of the present invention, a method for evaluating potential treatments for activity against prions or prion-related diseases is provided. The method includes subjecting a prion model to a treatment designed to attack prions, the treatment including:
cleaning the substrate with an alkaline cleaning composition which has an alkali concentration of 0.02 to 0.1 M and which removes proteins, and contacting the cleaned substrate with an oxidizing agent containing peracetic acid;
the prion model being an ileal fluid dependent organism (IFDO) model, which has been shown to exhibit a response similar to that of prions to a treatment designed to attack prions and evaluating the effect of the treatment on the prion model as an indicator of the effect of the treatment on the prion or prion-related disease.
The method is suitable for screening proposed drugs for activity against prion related diseases or proposed treatment processes or chemicals for priocidal activity. The method includes exposing a prion model to the proposed
drug, chemical, or process and culturing any remaining prion model *in vitro,* the prion model having been shown to exhibit similar responses to prions to a drug, chemical, or process.

One advantage of the present invention is that proposed prion disease treatments, pharmaceuticals, and priocidal agents can be screened *in vitro*, without the need for extensive *in vivo* study.

Another advantage of the present invention is that proposed prion disease treatments and priocidal agents can be evaluated rapidly.

still further advantages of the present invention will become apparent to those of ordinary skill in the art upon reading and understanding the following detailed description of the preferred embodiments.

### Brief Description of the Drawings

The invention may take form in various components and arrangements of components, and in various steps and arrangements of steps. The drawings are only for purposes of illustrating a preferred embodiment and are not to be construed as limiting the invention.
FIGURE 1 is a schematic side view of a biological indicator containing a prion model according to the present invention;
FIGURE 2 is a schematic side view of the biological indicator of FIGURE 1 after closing a cap to seal the indicator and mix the prion model with growth media;
FIGURE 3 is a plot of prion model count against time for peracetic acid treatment processes at different initial concentration levels of peracetic acid;
FIGURE 4 is a plot showing the total number of red blood cells over time for a first control sample (water-RBC), a second control sample (pancreatin-Panc) and a sample of the prion model (labeled IFD0);
FIGURE 5 is a plot of percent red blood cells having a normal structure (i.e., free of abnormal structure or aberrations) over time for a first control sample (water-RBC), a second control sample (pancreatin-Panc) and a sample of the prion model (labeled IFD0).

### Detailed Description of the Preferred Embodiments

An unusual proteinaceous entity which will be referred to herein as the "test protein" or "prion model" has been found to correlate with prion activity and is thus useful as simulated prion model. The prion model has been developed as an indicator for prion inhibition/inactivation and has shown correlation to prion inactivation in vitro and *in vivo.*

The preferred test protein was originally isolated by D.W. Burdon, Department of Microbiology, Queen Elizabeth Hospital, Birmingham, England. It was originally extracted from ileal fluid from patients suffering from Crohn's disease and is proteinaceous in nature. It appears to have associated iron and may have an RNA component. Microscopic examination at 100x shows the prion model to comprise pleomorphic particles of no uniform size or shape. We have subsequently shown this agent to be present in blood and serum samples.

It is contemplated, however, that other similar proteins, which show the same or similar resistance to destructive treatments as the present test model, may alternatively be used as the prion model.

The test protein is readily cultured in an artificial medium, such as agar-based media or liquid media. It can be used in the medium or suspended in water or other fluid. The protein appears to grow effectively when lysed red blood cells are present, thus it is preferable that the media contain red blood cells from humans or other animals, or extracts therefrom, such as hemoglobin. Since the prion model has been found in a sizeable proportion of blood samples, it is preferable for the blood to be screened prior to use in the growth medium to avoid any competing reactions *in vitro* and contamination of samples later to be cultured *in vivo*.

Very few actives have to date been shown to be effective at inhibiting the growth of the prion model in culture media. Of those found to have an effect, a number of these have previously been shown to have some activity against prions including vCJD, showing the correlation of the model with actual prion activity. These effective actives include sodium hydroxide (about 1M NaOH) and a phenol-based formulation sold under the tradename LpH™ by STERIS Corporation, Mentor, Ohio. A peracetic-acid based formulation, STERIS 20™, has previously been found to have activity against prions and has now been found to have activity against the prion model. Other actives with measurable activity, which have now been identified using the prion model, include nisin, neomycin sulphate, silver nitrate, and manganese.

Because of the strong correlations which have been found between the response of prions to various treatments and the corresponding response of the present test protein, the test protein can be used *in vitro* as a model to screen large numbers of potential drugs, priocidal agents, processes, and the like (all generally referred to as "treatments"), for their potential effect against prions and prion diseases. Treatments which prove to be effective under *in vitro* studies with the prion model can then proceed to *in vivo* testing, for example, by inoculating an animal with a prion and subjecting the animal to a treatment regimen (*e.g.*, in the case of a proposed drug for treating a prion disease), or by exposing a prion sample to a selected treatment (such as a proposed priocidal agent for use on medical instruments) and then culturing the exposed sample *in vivo*, to determine whether the prions have been destroyed by the selected treatment.

Using the prion model, conventional anti-microbial treatments (*e.g*., peracetic acid, steam, or vapor hydrogen peroxide) can be evaluated for their effectiveness against prions. Modifications to the conventional antimicrobial treatments can be made with a view to optimizing the anti-microbial treatment so that the treatment destroys or otherwise inactivates prions and also destroys or otherwise inactivates other microorganisms typically destroyed by such processes. The modified process can then be used to treat devices contaminated with both prions and with microorganisms. Novel actives, specifically designed for treating prions, can also be evaluated.

Another use for the prion model is to develop treatments for food products, generally those containing an animal product, which may be contaminated with prions. Such products include, for example, raw meat, including meat carcasses, ground meat, or chopped meat products, and cooked meat products, such as sausages, hams, processed meat products and the like. Using the prion model, several treatments have been identified. A method of treating a food product for animal or human consumption which may be contaminated with prions has therefore been developed. The method includes treating the food product with a composition which includes at least one of nisin, manganese, and silver nitrate in a sufficient concentration and for a sufficient time to reduce the level of prions on the food product or to inactivate the prions present. Animal products which are rendered or otherwise prepared for use as animal feedstocks may also be treated with the composition, either before or after rendering.

A method of treating items, such as medical or dental instruments, which may be contaminated with prions has been developed. The method includes treating the items with a composition which includes at least one of nisin, manganese, and silver nitrate to reduce the level of prions on the items. The concentration of the active in the composition and the length of treatment will depend on the type of active and degree of reduction sought. The treatment may be combined with other treatments known to reduce prions, such as steam autoclaving and/or sodium hydroxide or sodium hypochlorite treatment.

The composition for food or instrument treatment is preferably in liquid form, *e.g*., an aqueous solution of the active, although dry and non-aqueous compositions are also contemplated. Treatment may include, for example, immersion of the items to be treated in the treatment solution, spraying or otherwise contacting the items with the solution, or exposing the items to a vapor containing the active.

Another treatment method which has been developed using the prion model is to treat items which may have been contaminated with prions with oxidizing agent-based gaseous or liquid processing. As an example, a peracetic acid based solution is used. The method is useful for treatment of medical and dental instruments, and the like, particularly those which have been used for brain or related surgeries. Temperature has been found to have a significant effect on priocidal activity. The peracetic acid solution is preferably at a temperature of from 45-60°C, more preferably, from 53-57°C. It is postulated that a reduction in activity at higher temperatures may be due to coagulation of the protein, rendering it less accessible to the peracetic acid. The temperature range is also effective for destruction of other contaminants, such as microorganisms. This allows the same process to be used for both sterilization and prion reduction or elimination. The peracetic acid solution preferably contains buffers, surfactants, chelants, and may also contain anticorrosives for reducing damage to the instruments or to the treatment system. Surfactants are thought to be particularly important in the formulation, since they may affect the conformational structure of the prion model protein and allow the peracetic acid to be more effective. A peracetic acid concentration of at least 2000 ppm is preferred for rapid prion treatment, more preferably, about 2500 ppm. Peracetic acid concentrations in the range of 2000 to 2500 ppm have been found to break down the protein to a form which is inactive (small peptides or amino acids). Lower temperatures and concentrations are generally less effective. Higher temperatures may be damaging to temperature-sensitive devices and also reduce the half life of peracetic acid.

The peracetic acid treatment can be used to replace conventional, aggressive prion treatments such as steam/NaOH treatments. Or, the peracetic acid treatment can be used in combination with other treatments. One proposed treatment regiment includes:
1. Cleaning the instruments with a cleaning agent which has been found to be effective at removing proteinaceous material, particularly prions;
2. Heat treatment with steam or high temperature (*e.g*., 180°C) thermal rinse (optional, or may be carried out after step 3.);
3. Sterilization treatment, *e.g*., with a peracetic acid formulation at 2000 ppm, or above and a temperature of 55-57°C for 10-30 minutes.

The cleaning agent is an alkaline cleaning agent. Tests with the prion model have shown an increase in the effectiveness of the treatment process with the alkalinity of the cleaning product. Highly alkaline products, such as sodium or potassium hydroxide-based products, *e.g.*, CIP™ 100 (obtained from STERIS Corp., Mentor, OH) have been found to be particularly effective. Even though termed "highly alkaline," these products have a much lower alkali concentration (CIP™ 100 at 2 oz/gal contains 0.07 M KOH) than the 1N NaOH currently being recommended as a treatment for prions. Thus, a combination of an alkaline cleaning product wash at an alkali concentration of 0.02M to 0.1M followed by a peracetic acid treatment is an effective alternative to a treatment with 1N NaOH and is less damaging to the medical instruments or other items being treated.

The method for screening proposed drugs for activity against prion related diseases or proposed treatment processes or chemicals for priocidal activity includes exposing a prion model to the proposed drug, chemical, or process and culturing any remaining prion model *in vitro.* The prion model is one which has been shown to exhibit similar responses to prions to other actives and processes.

The method for evaluating the effect of a proposed treatment, drug, or other active against prions involves exposing the prion model to the proposed treatment, drug, or other active. After exposure, the prion model (or whatever remains viable) is grown in a suitable growth medium.

For example, in the case of a treatment process, such as a sterilizing process, a coupon or instrument is contaminated with the prion model and exposed to a sterilizing or cleaning treatment. A swab, sample, or extraction is taken after the treatment and is placed in a growth medium. If growth of the prion model is observed, the sterilizing or cleaning treatment has not been fully effective at destruction or removal of prions.

In the case of an evaluation of a potential active, a solution of the drug or other active is mixed with the prion model. After a selected exposure time, an aliquot of the solution is taken and cultured in the growth medium. Prior to culturing, the aliquot is preferably neutralized with a suitable neutralizing agent to inactivate the drug or other active under test. If the drug or active proves effective against the prion model, it can be used in an effective amount to treat a subject, such as a contaminated surface or a person or animal suffering from a prion related disease.

In one embodiment, effects on the growth medium are used as an indicator of residual prion model (and hence, by inference, of prion) activity. The growth medium preferably contains hemoglobin, either in a pure or relatively pure form, or mixed with other blood related products. For example, the growth medium may contain lysed whole blood or lysed red blood cells. If any of the prion model has survived the treatment process or exposure to the selected active, the hemoglobin content of the growth media is reduced as the prion model "grows." This can be observed by a reduction in the deep red color of the media, or by other detection techniques, such as chemical analysis, colorimetry, spectroscopy, or the like. If the media is a solid media, *e.g*., an agar gel, the color change can be observed as a ring around the prion model, which increases in size over time. Thus, destruction/inactivation of the model may be measured in term of the size of the hemoglobin ring at a particular time after the start of the culturing process. Or, as with liquid media, a change in color of the media may be used as an indication of the amount of prion model remaining.

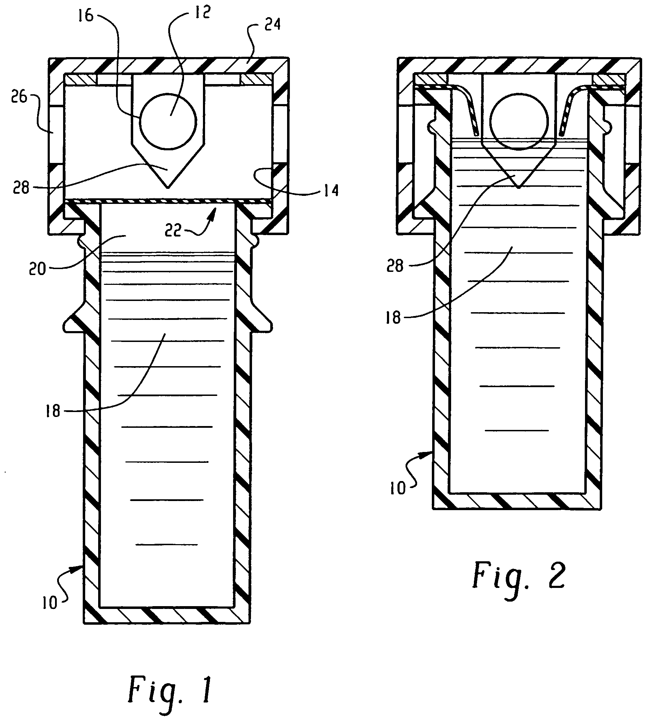

In one embodiment for testing a proposed treatment process (such as an oxidizing agent-based process, e.g., one using hydrogen peroxide or a peracid alone or in combination, in liquid or vapor form) for destroying prions on medical instruments or other devices, a prion model is contained in a biological indicator of the type conventionally used to test a sterilization or disinfection process for activity against selected, usually relatively hard to kill, microorganisms. One such indicator is exemplified in **FIGURE 1**, although other known biological indicator designs may alternatively be used. The indicator includes a vessel **10**, which contains a sample **12** of the prion model, preferably contained within the vessel such that it is not readily washed or otherwise removed from the vessel during the treatment process. The exact mode of containment will depend on the type of treatment process. In the case of liquid sterilants, such as peracetic acid, the prion model is contained within a protein impermeable barrier, which is permeable to the liquid sterilant. For gaseous sterilants, such as vapor hydrogen peroxide, or vapor peracetic acid, the prion model may be simply deposited on an interior surface **14** of a wall of the container or supported on a carrier **16**, such as a disk of paper, stainless steel, or polyflex. A suitable growth media may be added to the indicator just before subjecting the indicator to the proposed treatment process. More preferably, the prion model is mixed with a suitable growth media after testing. In one embodiment, a growth media **18** is contained in a penetrable portion or frangible ampule **20** within the vessel and then mixed with the prion model after removal of the indicator form the treatment process. One way to mix the growth medium and the prion model is to break the ampule or penetrate a wall **22** of the portion by movement of a cap **24** from an open position, shown in **FIGURE 1**, in which the sterilant has access to the vessel through openings **26**, to a closed position, shown in **FIGURE 2**, in which the cap seals the vessel, preventing exit and entry of fluids. In the embodiment of **FIGURES 1** and **2**, the cap has a projection **28** which pierces the wall **22**. Growth of remaining prion model is observed, for example, by a color change of the media or other detectable change in a physical or chemical property of the media. The color change may be due, for example, to production of the prion model (which appears blackish), reduction in the hemoglobin content of the media, or may be a result of an interaction between the prion model and a chemical indicator present in the media. An observed change in color is indicative that the proposed treatment process would not been effective as a treatment process for prion contaminated devices or other items.

In one embodiment, the indicator contains both a sample of the prion model and a sample of a microorganism which is known to have a high resistance to the type of treatment process under investigation. After subjecting the indicator to the proposed treatment process, the indicator is evaluated for residual prions and/or microorganisms. Treatment processes which are effective against both prions and microorganisms can thus be developed or optimized.

### Growth Media

A suitable growth media has been developed for culturing the prion model. The growth media contains an agar or broth base, such as Mycoplasma™ agar or broth base, obtained from Oxoid. A source of hemoglobin, such as washed and lysed red blood cells from a human or other animal, is also present. As discussed above, the red blood cells are preferably tested to make sure that no prions are present in the growth media prior to use. The media also preferably contains a source of proteases. The source of proteases may be an isolated protease, an enzyme extract, which may also contain other enzymes, such as lipases, or a comminuted animal tissue, such as pancreatin, which is obtained by homogenizing pancreas tissue. A dispersing agent, such as Tween 80, is preferably present. Thallium acetate is an optional component for reducing media contamination. The ingredients are blended with water, preferably distilled or other purified water.

The following exemplary growth media has been developed for use with the test protein:

| | |
|---|---|
| Distilled water | 1000mL |
| agar or broth base | |
| (*e.g*., Oxoid Mycoplasma™) | 10-100g |
| Dispersant (*e.g*., Tween 80) | 0-5mL |
| Washed and lysed horse red blood cells | 10-40 mL |
| Horse serum | 0-10mL |
| 0.1g/mL Pancreatin | 10-40mL |
| 2% thallium acetate | 2-20mL |

To test the "viability" of the prion model (*e.g.*, after a proposed treatment process), an aliquot of a culture or dilution containing the prion model is inoculated into a known volume of the above culture media and incubated at around 37°C for about 48-72 hours. The test proteins will "grow" as a black precipitate in the liquid culture or as discrete 'colonies' under the surface of a culture media plate. Although the prion model is not an organism, as is conventionally understood, the prion model does increase in amount on culturing and hence the term "grow," and similar terms, are used herein to denote an increase in the prion model. The term "viability" is used to denote prion model capable of exhibiting growth, i.e, it has not been destroyed or otherwise inactivated.

To prepare for in vitro tests, liquid cultures of the test protein are rapidly spun down at about 5000xg for about 5 minutes and washed in water or fresh media.

Testing of the simulated priocidal activity can be performed similar to typical tests with bacteria or fungi. These methods include:
1) Minimum inhibitory concentrations (MIC): this method allows for the rapid evaluation of a wide variety of actives to inhibit the culturing of the test protein. In a simple set-up with a microwell plate, serial dilutions of an active are performed and a standard low concentration (*e.g*., <10⁵ entity forming units (efu's)) of the prion model in growth media are added to each dilution. Following incubation at 37°C for 48-72 hours, the presence of growth is indicated by a black precipitate at the base of a well and the lowest concentration of an active to inhibit the growth is recorded as the MIC. This method may be used to identify possible drug targets or biocides for anti-prion activity.
2) Time kill experiments: this method allows for the determination of active or formulation efficacy over time. A test material (*e.g.,* a liquid formulation, product, or active) is prepared at a variety of concentrations and under a variety of environmental conditions (*e.g.,* pH, temperature, presence of a soil, and the like). The prion model culture, at a known concentration, is then directly added to the test liquid and aliquots removed over time for evaluation. The aliquots are preferably neutralized to inhibit further activity of the test material. Evaluation may include serial diluted of the aliquot and plating on selective media to determine the reduction of the test protein over time under the selected test conditions.
   In another test, the culture containing the test protein may be inoculated onto a substrate and exposed to a liquid or gas phase active for selected time exposures. Recoverable test protein is determined by serial dilution and plating.
3) Protein Degradation Studies. Protein containing media (either the test protein or another protein such as one having a high proportion of β sheet structure, similar to prion protein) are subjected to a treatment procedure, such as a peracetic acid treatment, and then aliquots are evaluated by gel electrophoresis or other technique capable of separating out complete proteins from smaller fragments. Several effective priocidal agents have been found to break down the prion protein into smaller fragments. Thus, agents which break down the test protein or other proteins can be viewed as potential prion-treating agents.

Such tests are valuable for optimizing the effectiveness of a given formulation, active, or process against the test protein. This is particularly important in understanding the effects of formulation and environmental factors on the activity of actives/biocides against prions. Preferably, more than one test is carried out, for example, a time kill study and a protein degradation study are carried out for the proposed treatment before proceeding to evaluate the proposed treatment on prions themselves.

The following examples indicate the effectiveness of the prion model as a model for actual prions and on the effectiveness of various proposed priocidal treatments.

### Examples

### Example 1: Growth Media

The following growth media was prepared:

| | |
|---|---|
| Distilled water | 1000mL |
| Oxoid Mycoplasma™ agar or broth base | 35.5 g |
| Tween 80 | 2mL |
| Washed and lysed horse red blood cells | 20mL |
| Horse serum | 1.4mL |
| 0.1g/mL Pancreatin | 20mL |
| 2% thallium acetate | 7mL |

When the test protein is inoculated on to a supplemented mycoplasma broth base plated media dish with the formula listed above, and cultured at 37°C, the inoculated spots yield discrete brown colonies on after 48 hours, under aerobic, microaerophilic or anaerobic conditions. No growth is observed at 4°C.

### Example 2: Composition of the test protein

The test protein was analyzed and determined to contain amino acids, as noted below, and at least two peptides.

When subjected to ICP, primarily iron was observed (although background calcium was seen, presumably all from the media).

### Total amino acid analysis

Cultures of the prion model were growth in broth, vigorous washed (by vortexing in saline 5 times) and dried. Samples were submitted for total amino acid analysis. Samples were hydrolyzed for 26 hours in 6N HCl at 110°C, dissolved in 0.01N HCl and analyzed by chromatography.

The results showed the presence of amino acids, with the following percentages:

| | |
|---|---|
| ASP | 8.30% |
| THR | 3.36% |
| SER | 9.08% |
| GLU | 7.38% |
| PRO | 5.24% |
| GLY | 10.96% |
| ALA | 7.11% |
| VAL | 5.97% |
| ILE | 2.41% |
| LEU | 12.14% |
| TYR | 1.61% |
| PHE | 3.60% |
| LYS | 5.84% |
| HIS | 11.26% |
| ARG | 5.70% |

### Protein analysis

The prion model protein was solubilized and protein gels were run on the supernatant. SDS-PAGE was used to separate the protein. The presence of a diffuse protein band was observed above the dye front (<10kDa). This band was transferred onto a membrane by Western blotting and submitted to CCF, Molecular Biology Core for N-terminal sequencing analysis. The signal was weak but indicated two peptide sequences as follows:
K L L/D H/W Q S Q/L H K/M Q R F
I Q K H I L Q K/I M/L A L E

### Example 3: Correlation Studies

To demonstrate the effectiveness of time-kill tests, and to establish a correlation between the response of the test protein with that of known prions, the log reduction of test protein was determined using actives known to be effective against prions. Log reduction is the difference between the log of the original number of organisms present (in this case, the number of test proteins, or, alternatively, the concentration of test protein in the sample) and the log of the number remaining. Good correlations were found for such actives. Examples are given below:

### a) Peracetic Acid Studies

Specific Peracetic Acid formulations (STERIS 20™ obtained from STERIS Corp., Mentor Ohio) previously proposed as effective priocidal agents were found to be effective against the prion model. STERIS 20™ is a peracetic acid-based sterilant containing buffers, surfactants, chelating agents, and anticorrosives. The results of these tests show that the temperature of the formulation is an important factor. Temperature and active concentration were found to have a surprising and significant effect on both protein and prion inactivation.

### i) Protein degradation studies

Protein degradation studies were carried out by exposing samples of the prion model to a sterilant (such as peracetic acid) and to control solutions (*e.g*., water or tris-buffered saline, TBS). After exposure, the peracetic acid was neutralized immediately with sodium thiosulfate (STS) and the sample is separated by gel electrophoresis. Sodium decyl sulfate-polyacrylamide gel electrophoresis (SDS-PAGE) is effective as a protein separating medium. Further, HLC analysis has shown that a major component of the model is a short peptide structure similar to the simple amino acid structure of collagen. The separated proteins were transferred to nitrocellulose by the Western blot method. The presence of particular proteins, such as the prion model, was detected by immunoblotting with monoclonal antibodies specific to the protein and quantified by the percentage intensity of immunostained proteins. Peracetic acid, when used in the particular formulations tested (STERIS 20™), reduced the amount of whole protein, creating smaller peptides, which are regarded as inactivated for purposes of the prion model.

### ii) Plating Studies

The efficacy of the peracetic acid formulations under different temperatures and concentrations was studied by suspension testing, using the prion model. Peracetic acid formulations were prepared at 1000, 1500, and 2000 mg/L peracetic acid and maintained at about 50°C. An aliquot of the test protein suspension was directly added to each formulation and samples removed and quantified by serial dilution and plating into modified Mycoplasma agar.

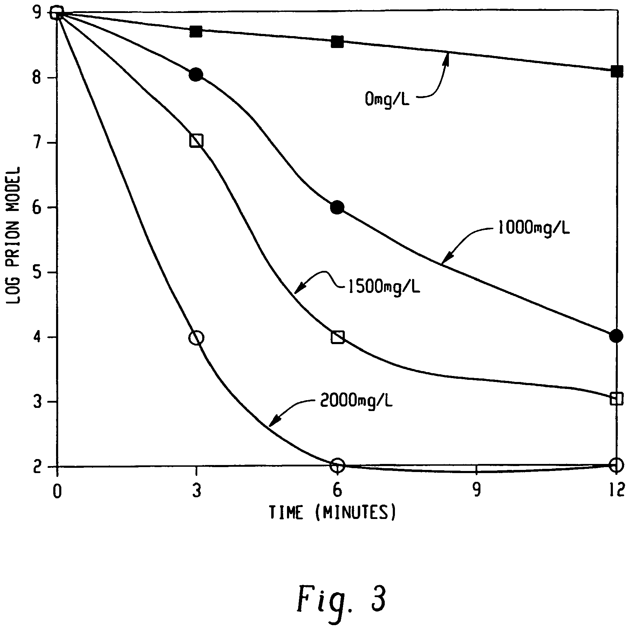

Time kill experiments were carried out to show the effect of time and peracetic acid concentration on the prion model. As an example, the effect of Peracetic acid concentration at 50°C was shown to be significant (**FIGURE 3**). Following incubation at 37°C for 48 hours, the plates were counted and log reductions determined. **FIGURE 3** shows the effect of Peracetic acid concentration (in mg/L) at 50°C for 12 mins in the STERIS 20™ formulation. At 2000 mg/liter, a log reduction from 9 log (initial concentration) to 2 logs was observed after 6 minutes.

For these tests, the peracetic acid was combined with the buffering system used in the STERIS 20™ formulation, the only differences being in the amount of peracetic acid employed.

These results are similar to a reported reduction of vCJD on Western blots with the same STERIS 20™ peracetic acid formulation (Antloga, et al., Prion Diseases and Medical Devices, ASAIO J., S69-S72 (2000). These results are being confirmed *in vivo.*

### b) Phenolic Formulation Studies

LpH™ is a phenolic formulation sold by STERIS Corporation, Mentor, OH. The composition has previously been described as being effective against scrapie in an *in* vivo study (Ernst & Race, Comparative Analysis of Scrapie Agent Inactivation Methods, J. Virological Methods, 41, pp. 193-202 (1993)). The authors described the loss of scrapie infectivity dependent on the concentration and time of exposure. For example, at a concentration of 0.9%, the infectivity removed, as measured by log reduction, was 5 logs after 0.5 hours and over 7 logs at 16 hours. Similarly, at 9%, an over 7 log reduction was observed at 0.5 hours.

The activity of the LpH™ product was tested on samples of the test protein at a 5% LpH™ concentration. The results indicated a 5.2 log reduction at 1 hour and a 5.8 log reduction at 3 hours. Further, the product was shown to be more effective than a similar product LpHse™. The same differences between LpH™ and LpHse™ which has also been shown by Race for prions (unpublished results).

### Example 4: Optimization of Peracetic Acid Treatment of Prion Model Samples

Testing using protein degradation as a screening tool has shown that a peracetic acid concentration of about 2000 mg/L or above is preferred for efficacy against the test protein **(****FIGURE 3**). These tests were carried out using STERIS 20™.

The effect of temperature on the test protein formulation (studied at 1000 mg/L peracetic acid) was found to be dramatic. The results preliminarily indicate that the peracetic acid formulation is highly effective at breaking down protein within the range of 50-57°C at the times tested in this study. Little to no breakdown was observed at temperatures below 50°C. At about 60°C, or above, similar loss in activity was observed. A temperature of about 55-57°C has been found particularly effective.

### Example 5: MIC investigations

The effects of a variety of actives (many with previous reports of possible effects on prions) in MIC (growth inhibition) tests on the prion model were studied.

The following actives showed at least some effect on growth characteristics of the prion model (i.e., a reduction in the growth of the prion model):
nisin (^{~}1000mg/L)
Klenzyme™(5%) (obtained from STERIS Corp.)
Renuklenz™ (5%) (obtained from STERIS Corp.)
NaOH (^{~}0.01N)
HCl (^{~}0.1N)
Peracetic acid (^{~}600mg/L)
neomycin sulphate (^{~}125 mg/L),
LpH™ (Obtained from STERIS Corp, Mentor, OH) (1-5%)
LpHse™ (Obtained from STERIS Corp, Mentor, OH) (1-5%)
Manganese (^{~}100 mg/L)
silver nitrate (^{~}30mg/L)

### Example 6: Removal Tests

A variety of cleaning agents were evaluated. Instruments were contaminated with Bovine Serum Albumin (BSA-a protein). To make the protein more difficult to remove, the instruments were heated at 110°C for one hour to denature the protein. The instruments were then washed in an automated washer using 1 oz./ gal. of a cleaning agent and a high wash temperature (150°C). After the washing cycle, visual examination for remaining soil was carried out. The cleaning agents evaluated are listed below in order of decreasing effectiveness. All the cleaning products were obtained from STERIS Corp., Mentor, OH.
CIP 100™ (a sodium hydroxide-based cleaner) -Most effective
CIP 150™ (a potassium hydroxide-based cleaner) Process Klenz™
Criti-Klenz™
Renu-Klenz™ (a neutral product)
CIP 220™ (an acid-based cleaner)
Water -least effective

The above order of effectivity also generally follows (with the exception of water) the alkalinity of the product. The most effective cleaning product, CIP 100™, also has the highest alkalinity. The least effective, CIP 220™, is acidic.

The same order of effectivity was found when the prion model was used in place of BSA.

### Example 7: Red blood cells viability assay

The prion model was found to adsorb/use hemoglobin or Red blood cells (RBCs) components in the growth media. A study of the effect of the prion model on whole red blood cells was carried out. RBCs were washed in saline 3 times and resuspended at a concentration to count cells under a Petroff Hauser at 40x (^{~}100/field). Total red blood cells and the percentage of cells which were still intact (unchanged in morphology) were monitored over time following incubation with the prion model (**IFDO**) or with water (**RBC**) or pancreatin **(Panc-this** had been shown in preliminary experiments to lyse RBCs).

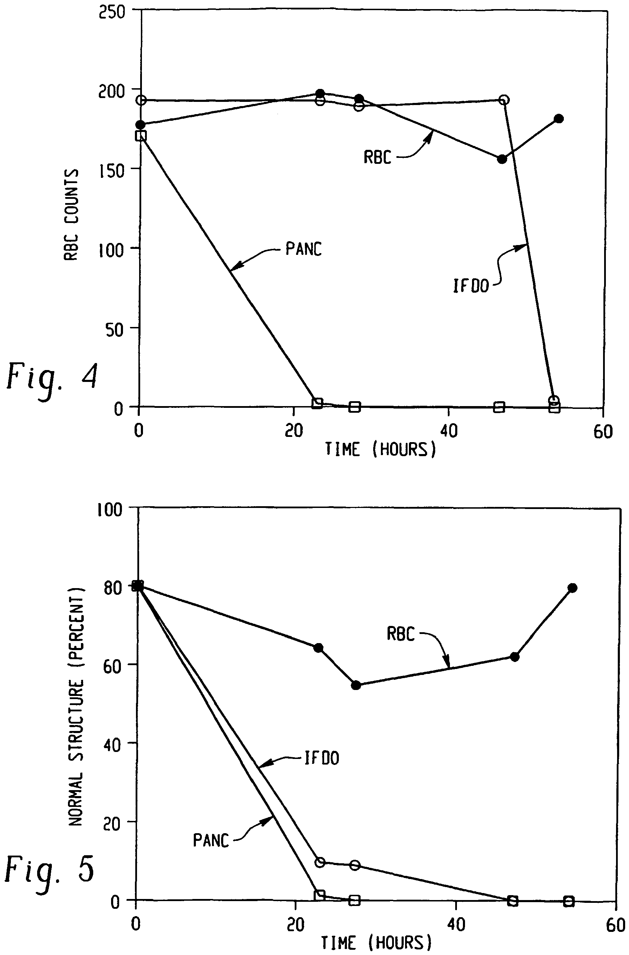

The results, shown in **FIGURES 4** and **5**, indicate that the prion model caused cell damage and lysis over time, when compared to the controls(**RBC** and **Panc**). There appears to be a lag time before red blood cells are lysed by the prion model.

### Example 8: Time kill experiments

Preliminary time kill experiments were conducted with the prion model. Results are expressed as log numbers (log₁₀), the lower the log number, the fewer the prions remaining and thus the more effective the treatment.

Time kill experiments were carried out to show the effect of autoclaving the prion model at 121°C in saline solution. The prion count dropped from 8.0 logs initially to 5.5 logs after 15 minutes.

Preliminary studies in a gravity drain cycle for 1 hour at 134°C showed the treatment was effective.

Time kill experiments were carried out to show the effect of ethylene oxide on coupons treated with the prion model. Coupons exposed at 600mg/L for 18 mins.

| | |
|---|---|
| | Prion count in Logs |
| Initial count | 8.5 |
| Exposure at 70°C, 100% RH | 5.0 |
| Exposure at 54°C, 40% RH | 4.7 |

As with peracetic acid, similar temperature effects were found, the ethylene oxide being more effective at 54 than at 70°C.

Liquid Disinfectants/Sterilants were evaluated in time kill studies for their effectiveness against the prion model. All of the products were obtained from STERIS Corp. Samples of the prion model in liquid suspension were directly inoculated into each product. The results are shown below.

| **Treatment** | **Time of Exposure (hrs)** | **Log No. of Prion Model** |
|---|---|---|
| Initial count | 0 | ^{~}6.6 |
| 10% CIP220™ | 1 | >3.0 |
| 10% CIP220™ | 3 | >3.0 |
| LpH™ | 1 | 1.4 |
| LpH™ | 3 | 0.8 |

### Example 9: Investigation of Intrinsic Contamination

Blood products were screened for contamination with prions. The following levels of contamination were found:
Horse blood (1 lot) contaminated
Horse serum (1 lot) contaminated
Bovine serum (3 lots) one lot contaminated
Sheep's blood (2 lots) none contaminated

The results indicate that prion contamination of blood products is common and therefore all blood products to be used in prion-related work should be screened for prions prior to use.

## Claims

1. A method for evaluating potential treatments for activity against prions or prion-related diseases, **characterized by**:
subjecting a prion model to a treatment, the prion model being applied to a substrate, the prion model comprising an ileal fluid dependent organism (IFDO) which has been shown to exhibit a response similar to that of prions to a treatment designed to attack prions, the treatment including:
cleaning the substrate with an alkaline cleaning composition which has an alkali concentration of 0.02 to 0.1 M and which removes proteins, and
contacting the cleaned substrate with an oxidizing agent containing peracetic acid; and
evaluating the effect of the treatment on the prion model as an indicator of the effect of the treatment on the prion or prion-related disease.

2. The method of claim 1, further **characterized by**:
the treatment including treating the prion model with a peracetic acid solution at a temperature of 50-58°C.

3. The method of claim 2, further **characterized by**:
the prion model being contained in a vessel which includes openings through which decontaminant has access to the vessel and a path by which the decontaminant contacts the prion model.

4. The method of any one of preceding claims 1-3, further **characterized by**:
the treatment including subjecting the prion model to a process known to be effective at destroying microorganisms.

5. The method of any one of preceding claims 1-4, further **characterized by**:
the treatment including treating a medical or pharmaceutical device which has been contaminated with the prion model with a composition which includes at least one of nisin, manganese, and silver nitrate.

6. The method of any one of preceding claims 1-5, further **characterized by**:
the step of evaluating including:
culturing remaining prion model in a growth medium; and
detecting growth of prion model.

7. The method of claim 6, wherein the step of evaluating includes:
growing the remaining prion model in a media containing hemoglobin, and
observing a change in color of the media as an indication of the amount of prion model remaining.

8. The method of claim 6 or 7, wherein the step of detecting includes subjecting the cultured prion model to a procedure which detects proteins.

9. The method of claim 8, wherein the step of detecting includes detecting protein fragments.

## Patentansprüche

1. Verfahren zur Bewertung potentieller Behandlungen auf Aktivität gegen Prionen oder mit Prionen zusammenhängenden Krankheiten, **gekennzeichnet durch**:
das Durchführen einer Behandlung mit einem Prionmodell, wobei das Prionmodell auf ein Substrat aufgetragen wird, wobei das Prionmodell einen von Ileumsaft abhängigen Organismus (IFDO) umfasst, von dem gezeigt wurde, dass er eine auf eine Behandlung, die Prionen angreifen soll, eine ähnliche Reaktion wie Prionen zeigt, wobei die Behandlung umfasst:
das Reinigen des Substrats mit einer alkalischen Reinigungszusammensetzung, die eine Alkalikonzentration von 0,02 bis 0,1 M aufweist und die Proteine entfernt; und
das In-Kontakt-Bringen des gereinigten Substrats mit einem Oxidationsmittel, das Peressigsäure enthält; und
das Bewerten der Wirkung der Behandlung auf das Prionenmodell als Indikator für die Wirkung der Behandlung auf das Prion oder die mit Prionen zusammenhängende Krankheit.

2. Verfahren gemäß Anspruch 1, weiterhin **dadurch gekennzeichnet, dass**:
die Behandlung das Behandeln des Prionmodells mit einer Peressigsäurelösung bei einer Temperatur von 50-58 °C umfasst.

3. Verfahren gemäß Anspruch 2, weiterhin **dadurch gekennzeichnet, dass**:
das Prionmodell in einem Gefäß enthalten ist, das Öffnungen, durch die ein Dekontaminant Zugang zu dem Gefäß hat, und einen Weg, über den der Dekontaminant mit dem Prionmodell in Kontakt tritt, umfasst.

4. Verfahren gemäß einem der vorstehenden Ansprüche 1 bis 3, weiterhin **dadurch gekennzeichnet, dass**:
die Behandlung das Durchführen eines Verfahrens, das bekanntermaßen die Zerstörung von Mikroorganismen bewirkt, mit dem Prionmodell umfasst.

5. Verfahren gemäß einem der vorstehenden Ansprüche 1 bis 4, weiterhin **dadurch gekennzeichnet, dass**:
die Behandlung das Behandeln einer medizinischen oder pharmazeutischen Vorrichtung, die mit dem Prionmodell kontaminiert wurde, mit einer Zusammensetzung umfasst, die wenigstens eines von Nisin, Mangan und Silbernitrat umfasst.

6. Verfahren gemäß einem der vorstehenden Ansprüche 1 bis 5, weiterhin **dadurch gekennzeichnet, dass**:
der Schritt des Bewertens umfasst:
das Kultivieren des verbleibenden Prionmodells in einem Wachstumsmedium; und
das Nachweisen des Wachstums des Prionmodells.

7. Verfahren gemäß Anspruch 6, wobei der Schritt des Bewertens umfasst:
das Wachsenlassen des verbleibenden Prionmodells in einem Medium, das Hämoglobin enthält, und
das Beobachten einer Farbänderung des Mediums als Hinweis auf die Menge des verbleibenden Prionmodells.

8. Verfahren gemäß Anspruch 6 oder 7, wobei der Schritt des Nachweisens das Unterziehen des kultivierten Prionmodells einem Verfahren, das Proteine nachweist, umfasst.

9. Verfahren gemäß Anspruch 8, wobei der Schritt des Nachweisens das Nachweisen von Proteinfragmenten umfasst.

## Revendications

1. Procédé d'évaluation de traitements potentiels concernant leur activité contre des prions ou des maladies à prions, **caractérisé par** :
la soumission d'un modèle de prion à un traitement, le modèle de prion étant appliqué sur un substrat, le modèle de prion comprenant un organisme dépendant du fluide iléal (IFDO) qui s'est avéré présenter une réponse à un traitement conçu pour attaquer des prions similaire à celle des prions, le traitement comprenant :
le nettoyage du substrat avec une composition de nettoyage alcaline qui présente une concentration en alcali comprise entre 0,02 et 0,1 M et qui élimine les protéines, et
la mise en contact du substrat nettoyé avec un agent oxydant contenant de l'acide peracétique ; et
l'évaluation de l'effet du traitement sur le modèle de prion en tant qu'indicateur de l'effet du traitement sur le prion ou la maladie associée au prion.

2. Procédé selon la revendication 1, **caractérisé en outre par** :
le traitement qui comprend le traitement du modèle de prion avec une solution d'acide peracétique à une température comprise entre 50 et 58 °C.

3. Procédé selon la revendication 2, **caractérisé en outre par** :
le modèle de prion qui est contenu dans une cuve qui comprend des ouvertures par lesquelles le décontaminant à accès à la cuve et une trajectoire par laquelle le décontaminant entre en contact avec le modèle de prion.

4. Procédé selon l'une quelconque des revendications 1 à 3, **caractérisé en outre par** :
le traitement qui inclut la soumission du modèle de prion à un procédé connu pour être efficace pour détruire les microorganismes.

5. Procédé selon l'une quelconque des revendications 1 à 4, **caractérisé en outre par** :
le traitement qui comprend le traitement d'un dispositif médical ou pharmaceutique qui a été contaminé par le modèle de prion avec une composition qui comprend au moins un élément parmi la nisine, le manganèse et le nitrate d'argent.

6. Procédé selon l'une quelconque des revendications 1 à 5, **caractérisé en outre par** :
l'étape d'évaluation qui comprend :
la culture du modèle de prion restant dans un milieu de culture ; et
la détection de la croissance du modèle de prion.

7. Procédé selon la revendication 6, dans lequel l'étape d'évaluation comprend :
la croissance du modèle de prion restant dans un milieu contenant de l'hémoglobine et l'observation d'un changement de couleur du milieu en tant qu'indication de quantité de modèle de prion restant.

8. Procédé selon la revendication 6 ou 7, dans lequel l'étape de détection comprend la soumission du modèle de prion cultivé à une procédure qui détecte les protéines.

9. Procédé selon la revendication 8, dans lequel l'étape de détection comprend la détection de fragments de protéines.
